# EUROPEAN PATENT SPECIFICATION

(11) **EP 4 111 196 B1**
(45) Date of publication and mention of the grant of the patent: **14.02.2024**
(21) Application number: 20819883.8
(22) Date of filing: 25.11.2020
(51) Int. Cl.: G01N 33/543, B01L 3/00, G01N 33/558

(54) **SAMPLE TEST CASSETTE AND ANALYTE TEST SYSTEM UTILIZING THE SAME**
PROBENTESTKASSETTE UND ANALYTTESTSYSTEM DAMIT
CASSETTE DE TEST D'ÉCHANTILLON ET SYSTÈME DE TEST D'ANALYTE UTILISANT LE MÊME

(30) Priority: 28.02.2020 DK PA202000257
(43) Date of publication of application: 04.01.2023
(73) Proprietor: FOSS Analytical A/S, 3400 Hilleroed (DK)
(72) Inventor: BORN, Christian, 3400 Hilleroed (DK); ABBONDIO, Allan Bjerre, 3400 Hilleroed (DK); MATTHIESEN, Steen Hauge, 3400 Hilleroed (DK)
(86) International application number: PCT/IB2020/061131
(87) International publication number: WO 2021/171077

(56) References cited:
- WO-A1-2005/045408
- WO-A1-2017/143323
- US-A1- 2015 160 208
- US-A1- 2018 304 260

## Description

The present invention relates generally to the detection of one or more analytes in a sample liquid using lateral flow test strips and includes a sample test cassette therefor, as well as an analyte test system utilizing the same.

The detection of analytes in a sample liquid using an immunoassay based device employing a lateral flow test strip (also often referred to as a lateral flow device or LFD) is well known. Many of these immunoassay based devices include a rigid housing encasing an elongate lateral flow test strip of known type. One such immunoassay based device is described in US 9,833,783 and comprises a cassette formed internally with at least one elongate channel for locating therein an elongate lateral flow test strip orientated with one end in liquid communication with a liquid flow channel. A liquid receiving void is provided as an inlet for receiving a sample liquid and is in liquid communication with the flow channel at a location upstream of the at least one elongate channel. Sample liquid is pipetted into the liquid receiving void by a user and is transported under gravity to contact the end of the test strip which is in liquid communication with the liquid flow channel. Once contacted with the end of the test strip liquid flows laterally along the element by capillary flow and either any analyte therein, or a complex thereof, or some other reagent in the test strip interacts with suitable capture agents bound at one or more test zones of an analysis region of the test strip to thereby produce a detectable signal. An inspection of the analysis region is made either visually or with a reader to determine the presence of analyte in the liquid. Control zones may also be included in the analysis region and similarly inspected to determine the correct operation of the test strip or to aid in the quantitative determination of analyte in the liquid.

According to a first aspect of the present invention there is provided a sample test cassette as defined in claim 1 comprising an inlet for introducing a sample liquic into the sample test cassette; and one or more elongate channels, each for receiving an elongate lateral flow test strip and each configured with a first end in liquid communication with the inlet; wherein the sample test cassette further comprises an integral mechanical transport system adapted to generate a flow of sample liquid from outside of the inlet and to the first end of each of the one or more elongate channels. The integral mechanical transport system allows for the introduction of sample liquid to each lateral flow test strip received in the elongate channel(s) in a controllable manner so that one or both the amount of sample introduced and the flow rate can be controlled and/or automated in a repeatable manner and a multiplexed test using multiple test strips may be initiated simultaneously. Such a sample test cassette may be used by essentially untrained operators with a reduced potential for operator-induced errors.

In some embodiments, the flow channel comprises a reservoir, such as may be provided by a well and/or a bibulous material, located in liquid communication with the first end of each of the one or more elongate channels. This has an advantage that an adequate volume of liquid may be retained for uptake by lateral flow test strips located in one or more of the elongate channels without the need to provide a continuous flow in the cassette.

In some embodiments the transport system comprises a piston pump having a variable volume pump chamber in fluid communication with the inlet.

In some embodiments at least a section of a wall of the sample test cassette that overlies at least a portion of each of the one or more elongate channels corresponding with an analysis zone of a lateral flow test strip received therein is adapted to allow transmission of optical radiation to and from the analysis zone. This permits detection of an analyte of interest by optical interrogation of the lateral flow test strips.

According to a second aspect of the present invention there is provided an analyte test system as defined in claim 6 comprising a housing; a reading system,
preferably an optical reading system; and one or more holders;
wherein each of the one or more holders is configured to releasably locate a sample test cartridge in a reading position, at which reading position the reading system is aligned all of the one or more elongate channels to permit an interrogation of each test strip located in the one or more elongate channels to test for the presence of an analyte in the sample, for example by detection of light after transmission through, reflection from or passive (fluorescence, say) or active (electrochemical luminescence, say) generation at, an analysis region of each test strip.

In some embodiments the reading system is an optical reading system which comprises an own light source and an own optical detector located internal of each holder. This permits movement of the holder, such as rotation of the holder into and out of the housing, whilst maintaining the alignment of the optical reading system so that interrogation may be made at different positons of the holder.

In some embodiments the analyte test system further comprises an actuator mechanism adapted to engage with the transport system of a sample test cartridge located in the holder and to actuate the transport system to generate the flow of liquid.

In some embodiments each holder holds internally an own actuator mechanism.

In some embodiments the actuator may comprise an electric motor and in other embodiments the actuator may comprise a wound spring driven motor where, usefully, the spring may be wound by the action of placing the sample test cassette in the holder or the holder in the housing.

These and other features and advantages of the present invention will now be further described with reference to, and will become apparent from, exemplary embodiments which are illustrated in the drawings of the accompanying figures, of which:
- Fig. 1: Illustrates a first embodiment of a sample test cassette;
- Fig. 2: Illustrates an elongate test strip of known type suitable for use in the sample cassette of Fig. 1;
- Fig. 3: Illustrates an analyte test system with a sample test cassette of Fig. 1;
- Fig. 4: Illustrates a holder of the analyte test system of Fig. 3;
- Fig. 5: Illustrates the operation of the actuator of the analyte test system according to Fig. 3;
- Fig. 6: Illustrates a further embodiment of an actuator; and
- Fig. 7: Illustrates a further embodiment of a transport system.

As used within this specifications, including in the claims, the singular articles "a"; "an" and "the" include the plural unless the context clearly indicates otherwise. The use of the phrases "one or more", "at least one" or similar phrases, does not alter the generality of the foregoing.

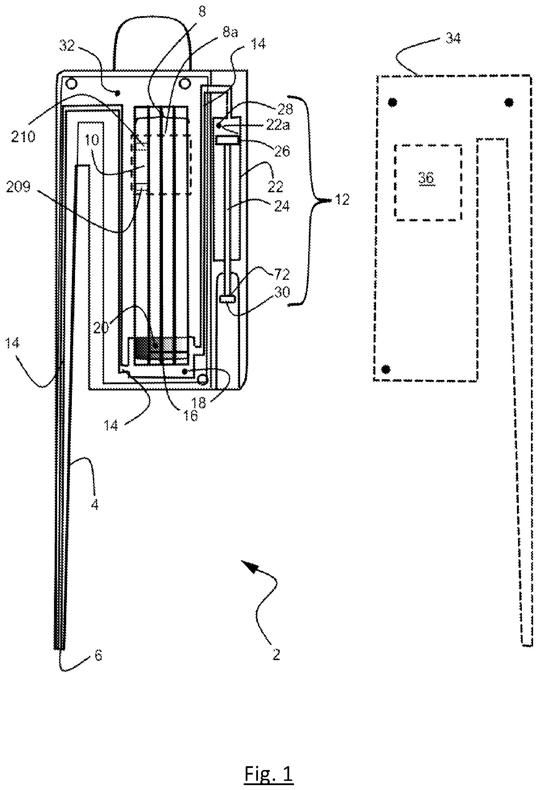

An example of a sample test cassette 2 according to the present invention is illustrated in Fig. 1. The sample test cassette 2 comprises an inlet 4 having an externally accessible opening 6 through which a sample liquid may pass into the cassette 2; one or more (illustrated 4) elongate channels 8 for retaining therein a respective elongate lateral flow test strip 10 (here one illustrated); and a mechanical transport system 12 which is made as an integral part of the cassette (2).

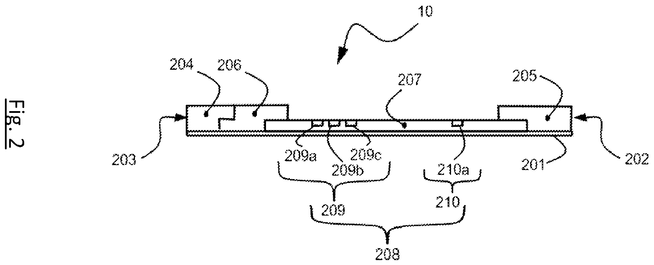

An example of an elongate lateral flow test strip 10 which is suitable for use in the sample test cassette 2 of the present invention is illustrated in Fig.2 and is of a generally known construction. The elongate lateral flow test strip 10 comprises a rigid elongate support 201 having a downstream end 202 and an upstream end 203. A sample pad 204 for receiving a sample liquid is affixed to the support 201 proximal its upstream end 203 and a waste pad 205 is affixed to the support 201 proximal its downstream end 202. A probe pad 206 is affixed to the support 201 in physical contact with the sample pad 204 and releasably holds probe elements which are designed to bind to and flow with specific analytes in the sample liquid. A porous membrane 207 is affixed to the support 201 and extends between and contacts the probe pad 206 and the waste pad 205. The porous membrane 207 has an analysis zone 208 which consists of one or more test zones (one shown 209) and one or more control zones (one shown 210). Each test zone 209 comprises one or more spatially defined test regions (here three shown 209a, 209b, 209c), which may be strips or points provided on the porous membrane 207, each region fixedly holds a same or different specific recognition elements (such as aptamers, receptor protein fragments or antibodies) which are selected to bind to specific analytes in a sample liquid. Each control zone 210 comprises one or more spatially defined control regions (here one shown 210a), which may be strips or points provided on the porous membrane 207, each region fixedly holds affinity ligands which typically binds probe elements which were originally contained in the probe pad 206. Typically, in use the sample pad 204 acts as a sponge to hold an excess of the sample liquid. Once the sample pad 204 is soaked, the sample fluid will flow from the sample pad 204 and into the probe pad 206 in which the probe elements are releasably stored. The sample fluid, including the probe bound analyte, flows from the probe pad 206 and along the elongate porous membrane 207 by capillary action to reach the test zone 209 where the probe elements of a specific test region 209a, 209b or 209c bind to and capture at least some of the probe bound analyte. The remaining liquid continues to flow in the elongate porous membrane 207 to reach the control zone 210 (placed downstream of the test zone 209 in the direction of flow of the liquid along the test strip 10) where probe elements remaining in the liquid are captured and bound and provides an indication that the test is working correctly. Liquid continues to flow in the elongate porous membrane 207 until it reaches the waste pad 205 which acts as a waste reservoir.

It will be appreciated that other, known, types of lateral flow test strip may be employed without departing from the invention as claimed, for example a lateral flow test strip generally as described above may be employed in which at least one of the sample pad 204, probe pad 206 and the waste pad 205 may be omitted.

Considering again Fig. 1, a conduit 14 connects the inlet 4 to a first end 16 of each of the elongate channels 8 and provides a liquid passageway for a sample liquid from external the opening 6 to each of the ends 16. In the present embodiment a reservoir 18 is provided connected to the conduit 14 and to the first ends 16 of the channels 8. The reservoir 18 provides a common source of liquid to each of the first ends 16 for uptake by a lateral flow test strip 10 retained in a respective elongate channel 8 and orientated with its sample receiving end, here the sample pad 204, positioned towards the first end 16 of the elongate channel 8 in which it is retained. In some embodiments (as illustrated in Fig. 1) a bibulous material 20 may be provided in or as the reservoir 18 for maintaining sample liquid in contact with the sample pad(s) 204. The conduit 14 also connects the first ends 16 (here illustrated as a connection via the reservoir 18) to the mechanical transport system 12. The mechanical transport system 12 operates to generate a flow of sample liquid from the outside of the opening 6, through the sample test cassette 2 and at least into the reservoir 18 in order to provide a source of sample liquid for uptake by the one or more elongate lateral flow test strips 10 that are each located in a respective elongate channel 8. It will be appreciated that in order to use the sample test cassette 2 it is not essential that all elongate channels 8 of the sample test cassette 2 contain a test strip 10. Moreover, it is not essential that each test strip 10 has the same number of test regions 209a, 209b, 209c and/or control 210a regions or that each test region 209a, 209b, 209c of different test strips 10 hold the same recognition elements. In some embodiments, each of the plurality of test strips held in a sample cassette may comprise only one test region but each test region holds a different recognition element. Thus multiple analytes may be readily and simply tested for using a same sample test cassette.

In the present embodiment the mechanical transport system 12 consists of a piston pump assembly which comprises a pump chamber 22 that is arranged in fluid communication with an end of the conduit 14; and a piston 24 having a first end 26 slidably engaged with an inner wall 22a of the pump chamber 22 to delimit therewith a variable volume fluid receiving space 28. A second end 30 of the piston 24 is also provided which is accessible external of the sample test cassette 2.

In some embodiments the maximum volume of the variable volume fluid receiving space 28 (i.e. when the piston 24 is at maximum extension) is selected to be approximately equal to the volume of liquid necessary to fill the reservoir 18. In this way an amount of sample liquid introduced into the sample test cassette 2 may be limited to that necessary for correct operation of the test strip(s) 10 without liquid being drawn into the sample receiving space 28.

A part of the sample test cassette 2 which overlies at least a portion 8a of each of the one or more elongate channels 8 that corresponds with an analysis zone 208 of a lateral flow test strip 10 when the test strip 10 is received therein is constructed to permit an external optical inspection of the test strip 10, in particular of the analysis zone 208 of the test strip 10. In the present embodiment this part is provided by a transparent wall section 32. By way of example only, the transparent wall section 32 may extend to also cover the conduit 14, the reservoir 18 and the entire length of the elongate channels 8. The transparent wall section 32 may be permanently bonded to the cassette to form a fluid tight cover after insertion of the elongate lateral flow test strip(s) 10 into corresponding channel(s) 8. Thus a disposable, one-time use, sample test cassette 2, may be constructed. This at least simplifies the formation of the flow conduit 14 which, instead of being constructed as a bore through solid material, may now be more simply and accurately constructed as a channel to be covered by the separate wall section 32.

In other embodiments, the transparent wall section 32 may be formed as a window covering essentially only the portions 8a of the elongate channel(s) 8 which will overlie the analysis zone(s) 208 of the test strip(s) 10, or may be omitted entirely and a solid wall section 34 provided to cover the flow conduit 14, the elongate channel(s) 8 and the reservoir 18 once the test strip(s) 10 are loaded into the elongate channel(s) 8. In such embodiments an aperture 36 is formed in the solid wall section 34 to overlie the portions 8a of the elongate channel(s) 8 that corresponds with the analysis zone(s) 208 and provides for external optical inspection of the analysis zone(s) 208. In some embodiments the transparent wall section 32 may be provided as part of a covering bonded to each of the lateral flow test strip(s) 10.

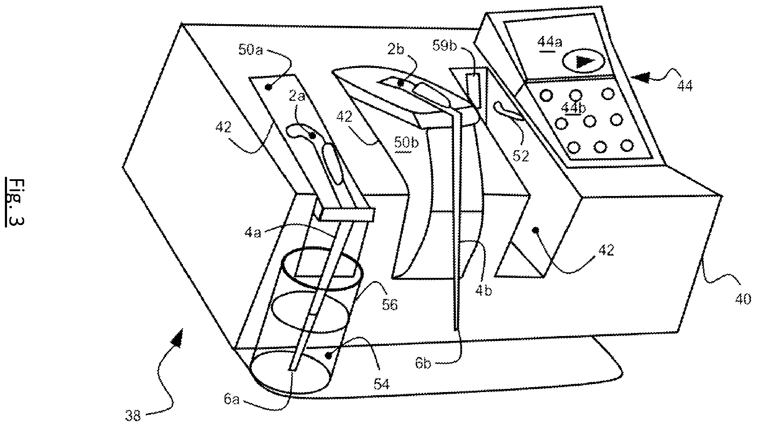

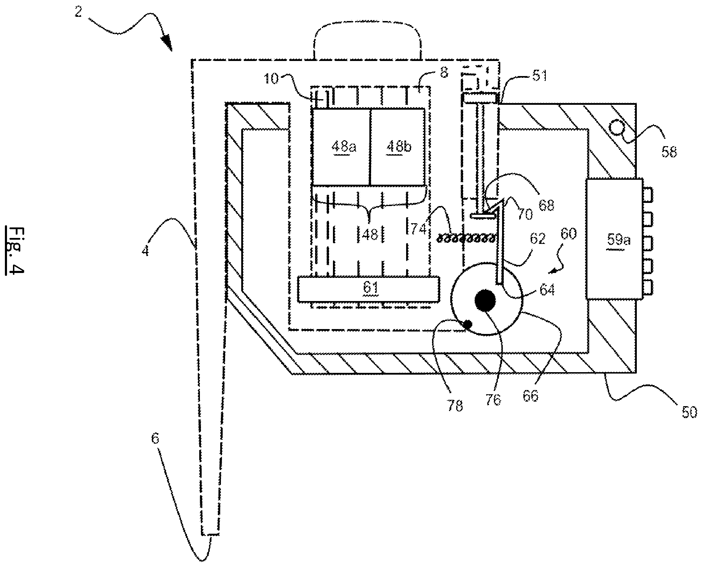

An analyte test system 38 which is suitable for use with a sample test cassette 2 described above is will now be described with reference to the illustrations contained in Fig. 3 and Fig. 4. The analyte test system 38 comprises a housing 40 having a number of slots 42 (here three) formed therein; a reading system 48; and a user interface 44 for inputting data into and/or for receiving data from the system 38. The user interface 44 is here illustrated as comprising a display, usefully a touch display region 44a, and a keypad region 44b by which a user can interact with the system 38. In some embodiments the user interface 44 may be incorporated, in whole or in part, in a smart device such as a smartphone or tablet computer. The analyte test system 38 may be powered from an external power source (such as mains supply); an internal power source (such as a battery) or both selectively. An optical reader (not shown) may usefully be incorporated into the housing 40 and may be configured to read a bar-code or QR-code which is associated with the cassette 2 and which may hold or point to information related to the test or tests to be performed by the one or more test strip(s) 10 which are housed in that cassette 2. Such information may be employed in the analyte test system 38 to control the operation of certain components of the system 38 in order to provide a test protocol specific to the sample test cassette 2.

The slots 42 are each adapted to releasably receive and hold a sample test cassette 2 in a reading position at which the optical reading system 48 is aligned in an optical path with the portion(s) 8a of the elongate channel(s) 8 corresponding with the analysis zone(s) 208 of the lateral flow test strip(s) 10 received therein. In the present embodiment each slot 42 is adapted to retain (usefully releasably) a holder 50 which, in turn, is adapted to releasably receive and hold a sample test cassette 2 in a cavity or slot 51 so that the sample test cassette 2 is held in the reading position internal of the holder 50 in the slot 51. In other embodiments each of the one or more slots 42 may be configured to receive and hold the sample test cassette 2 directly.

In order to provide a better understanding of the analyte test system 38 of the present invention, Fig. 3 illustrates a first holder 50a which is fully inserted into and retained in its corresponding slot 42; a second holder 50b which is partially inserted into its corresponding slot 42 and an empty slot 42 in which, in the present embodiment, can be seen a one of a pair of guide grooves 52. It is not essential that all slots 42 are filled with holders 50 in order to use the analyte test system 38.

In some embodiments, as illustrated in Fig. 3, when a holder (50a say) is fully inserted into a corresponding one of the slots 42 the open end (6a say) of inlet (4a say) of the sample test cassette (2a say) can be immersed in a sample liquid 54 in a sample vial 56. When a holder (50b say) is rotated in a corresponding the slot 42 the corresponding open end (6b say) of inlet (4b say) can be moved to permit removal of the vial 56 (and any sample liquid 54 it contains), for example for use of the remaining sample liquid 54 in other analysers, perhaps employing different analysis modalities, whilst the lateral flow analysis is still underway.

In some embodiments, as illustrated in Fig. 3 and Fig. 4, a holder 50 may be provided with outwardly protruding pins 58 which engage with, and here can rotate in, the guide grooves 52 of an empty slot 42 to allow a holder (50b say) to be inserted into and removed from the housing 40. In some embodiments rotation of the holder (50b say) in a slot allows an open end 6b of a sample cassette 2b held in the holder 50b to be moved into and out of contact with a sample liquid and thereby facilitate the introduction of a sample vial for sample testing.

An example of a holder 50 which forms a part of the analyte test system 38 of the present invention is illustrated in section in Fig. 4 and is equivalent to the holders 50a, 50b illustrated in Fig. 3. A sample test cassette 2 is also illustrated in Fig. 4 by the broken line construction in order to show its position relative to the components of the holder 50 when it is fully located in the holder 50.

The holder 50 of the present embodiment houses the optical reading system 48 and an actuator mechanism 60. In other embodiments one or both the optical reading system 48 and actuator mechanism 60 may be located external of the holder 50 and housed within the housing 40 of the analyte test system 38.

In some embodiments at least one electrical connector 59a is provided in the holder 50 to interface with a corresponding connector 59b located in a slot 42 of the housing 40 and thereby establish data, control signal and electrical power connections, as appropriate. A wireless communications unit, such as a known Bluetooth^{™} or WiFi enabled unit, may be included in the holder 50 for wireless transmission of data (including data from the optical reading system 48 and/or control signals) to and from the holder 50.

In some embodiments the at least one electrical connector may comprise a cable connector provided with an interface (such as sockets) to mate with a corresponding interface (such as pins) of a cable which terminates within the housing 40.

In some embodiments a temperature regulator 61 is also housed in the holder 50. The temperature regulator 61 may for example, comprise a Peltier heater/cooler element or a resistive heating element, together with, in some embodiments, a temperature sensor, and may be employed for incubation of the sample liquid prior to testing. The temperature regulator 61 is usefully made responsive to control signals sent via the interface 59a to maintain the sample cassette 2 (or relevant portions thereof) at a predetermined incubation temperature for a predetermined time. Such control signals may be generated in response to signals received from the temperature sensor, when present.

The reading system 48 is a one known in the art for use in reading elongate lateral flow test strips 10 and in the present embodiment is an optical reading system 48. In other embodiments the reading system may be an electrical capacitance or resistance reader of known type and the test strip(s) will be selected accordingly. The optical reading system 48 comprises a light source 48a and complementary detector 48b located at a position, in this embodiment inside the holder 50, in an optical path to permit optical interrogation of the analysis zone(s) 208 of test strip(s) 10 located in the sample test cassette 2 retained in the holder 50. Typically, and as is known, the optical reading system operates to detect optical changes which occur in the analysis zone(s) 208 of the test strip(s) as a result of interaction between components in the sample liquid flowing in the test strip(s) 10 and recognition elements in the one or more test region(s) 209a,b and/or c and in the one or more control region(s) 210a. It will be appreciated that an advantage of locating both the light source 48a and the detector 48b internal of the housing 50 is that the optical path permitting the optical interrogation remains invariant irrespective of the orientation of the holder 50 so that detection may be performed independently of the orientation of the holder 50 (even when a holder, 50b say, has been rotated, for example to allow removal of the vial 56).

Data from the detector 48b, representing optical information obtained from the analysis zone(s) 208, may be transmitted to external the holder 50, for example via interfaces 59a, 59b or via a wireless communications unit, for receipt by a data processor (not shown) which may be housed in the housing 40; or which may be located external of the housing 40, such as at a remotely located server, in communication with the system 38 via a wired or wireless communications link; or which may comprise elements located both internal the housing 40 and remote of the housing 40. However configured, the data processor is adapted, through suitable programming, to process the received data to detect changes that may have occurred in the analysis zone(s) 208 and therefrom to determine the presence of one or more analytes of interest in the sample liquid 54. The results of this determination may then be supplied for presentation on the display 44a of the analyte test system 38. The data processor may also be adapted to control the operation of the other elements of the analyte test system 38, such as control of the temperature regulator 61 and of the actuator mechanism 60.

The actuator mechanism 60 is operable to actuate the transport system 12 of a sample test cassette 2 held in the holder 50 to cause a flow of sample liquid (say sample liquid 54 held in vial 56 illustrated in Fig. 3) from external of the open end 6 of the inlet 4 for uptake by the sample pad(s) 204 of an elongate lateral flow test strip(s) 10 held in the cassette 2.

In some embodiments, the actuator mechanism 60 may comprise an arm 62 having a first end 64 pivotably mounted on a rotatable disc 66 and a detent 68 forming at least a part of a second end 70 for releasably mechanically engaging the transport system 12 at a surface 72 of the second end 30 of piston 24. The arm 62 is biased towards the piston 24, here by a spring bias 74, so that as the sample cassette 2 is entered into the holder 50 the detent 68 positively engages the surface 72. In some embodiments a motor (not shown) is also provided internal the holder 50 to impart rotary movement to a shaft 76 on which the rotatable disc 66 is mounted. In other embodiments the motor or both the motor and the shaft 76 may be located external of the holder 50, internal of the housing 40 of the analyte test system 38 to engage the rotatable disc 66 when the holder 50 is fully located in a corresponding slot 42 of the housing 40. In some embodiments a protrusion 78, such as a pin, is provided on the rotatable disc 66 at a location circumferentially displaced from the first end 64 of the arm 62.

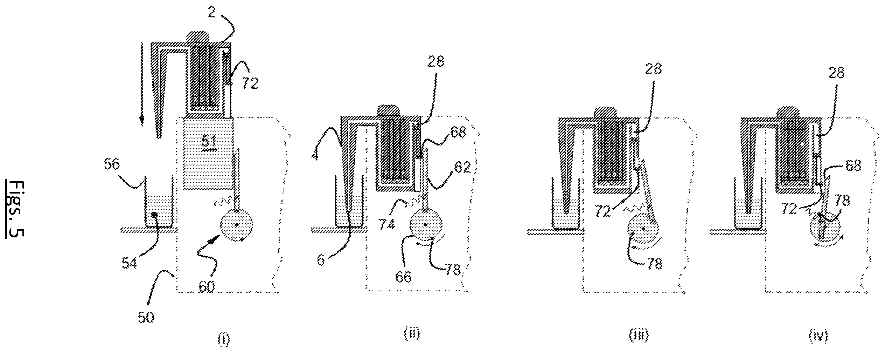

The operation of the actuator mechanism 60 will now be further explained with reference to the drawings of Figs. 5. the sample test cassette 2 is inserted into the holder 50 (Fig. 5(i)) until the opening 6 of the inlet 4 is immersed in sample liquid 54 in vial 56 and the detent 68 is engaged with the surface 72 of piston 24 (Fig. 5(ii)), to lock the cassette 2 in the holder 50 in its reading position. The arm 62 of the actuator mechanism 60 is now at or close to its highest position and the spring bias 74 maintains a positive contact between detent 68 and surface 72. The disc 66 is rotated (curved arrow in Fig. 5(iii)) to move the arm 62 in a generally downwards direction. This results in a corresponding downwards movement of the piston 24, causing an increase in the volume of the variable volume fluid receiving space 28 and an uptake of sample liquid 54 into the sample test cassette 2. The rotation of the disc 66 is continued and the protrusion 78 on the disc 66 engages the arm 62 (Fig. 5 (iv)). At this point the variable volume fluid receiving space 28 is at its maximum volume and transport of sample liquid 54 into the cassette 2 is completed. Typically now, rotation is halted and the optical reading system 48 (or other known reading system) is operated to interrogate, here optically, the test strip(s) 10 to determine the presence or absence of an analyte in sample liquid 54 that has been transported into the sample test cassette 2. The rotation of the disc 66 may then be continued. The protrusion 78 pushes against the arm 62 and causes the detent 68 to disengage from the surface 72. The sample test cassette 2 is now no longer locked in the holder 50 by the detent 68 and may be removed.

In some embodiments, the speed of rotation of the disc 66 may be variable in order to maintain a constant linear movement of the piston 24. This is useful in order to avoid cavitation in the sample liquid 54 which may produce undesirable bubbles in the sample liquid within the cassette 2. Indeed, any desired linear movement profile for the piston 24 may be achieved through suitable regulation of the rotation of the disc 66.

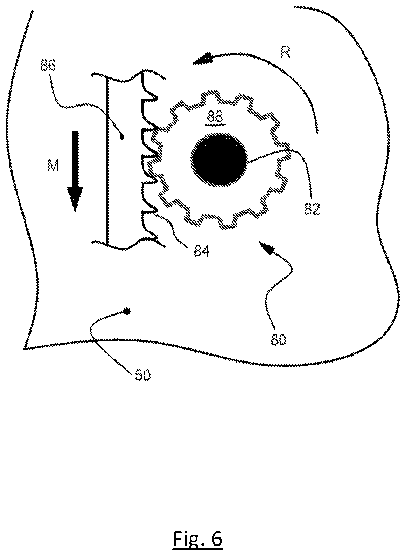

A further embodiment an actuator mechanism 80 is illustrated in Fig. 6 together with related portions of a transport system equivalent to the transport system 12 of the sample test cassette 2 which is illustrated in Fig. 1. Illustrated is a toothed portion 84 of a piston 86 of a piston pump assembly, similar to the piston pump assembly of the transport system 12 of the embodiment illustrated in Fig. 1. The actuator mechanism 80 comprises a sprocket 88 mounted on a rotatable shaft 82 of a motor (not shown). The sprocket 88 engages the toothed portion 84 as a sample test cassette is entered into the holder 50. Rotation of the sprocket 88 in one direction R causes linear movement M of the piston 86 to increase a volume of a variable volume fluid receiving space of the piston pump assembly and an uptake of sample liquid from external of the sample test cassette.

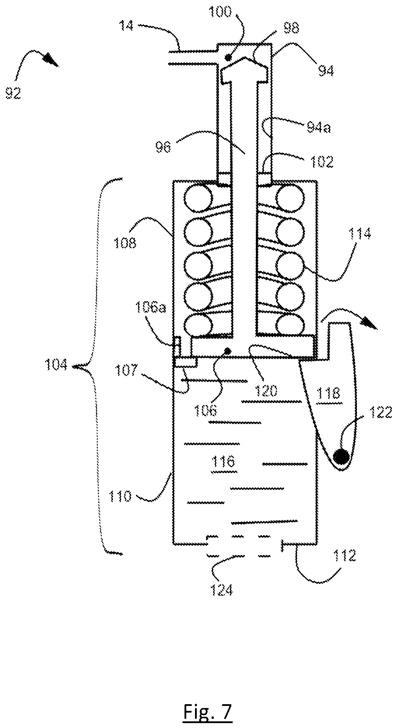

A further embodiment of transport system 92 is illustrated in Fig. 7 that may substitute for the transport system 12 which is illustrated in Fig. 1. Different to the transport system 12 of Fig. 1, and as will be described below, the present transport system 92 requires no external drive motor in order to maintain a flow of sample liquid within the sample test cassette of the present invention.

The transport system 92 comprises a pump chamber 94 that is arranged in fluid communication with an end of the conduit 14; and a piston 96 having a first end 98 slidably engaged with an inner wall 94a of the pump chamber 94 to delimit therewith a variable volume fluid receiving space 100. The piston 96 passes out of the pump chamber 94 through a fluid tight seal 102 into a compartment 104 where it terminates at a second end 106. The second end 106 provides a fluid tight seal and divides the compartment 104 into a spring chamber 108 and a damping chamber 110 which is sealed at an end 112 opposite the second end 106. The second end 106 is provided with a number of through holes (one illustrated 106a ) which provide a liquid passageway between the damping chamber 110 and the spring chamber 108 and each of which, in the present embodiment, are sealed by a pressure sensitive, rupturable seal 107. The spring chamber 108 houses a spring 114 under tension and provides a biasing force which acts on the second end 106 of piston 96 to tend to move the piston 96 to cause the variable volume fluid receiving space 100 to increase. A damping liquid 116 fills the damping chamber 110 and provides a hydraulic pressure which produces a force opposing but less than the biasing force of the tensioned spring 114. The spring 114 and damping liquid 116 cooperate to form an actuator mechanism. A latch 118 is provided to releasably engage the piston 96 and hold it against the bias force in a rest position. In the present embodiment the latch 118 locates against a lower surface 120 of the second end 106 of the piston 96 to prevent movement of the piston 96 until transportation of sample liquid into the cassette is required and is moveable to disengage from the piston 96, in the present embodiment by rotation about a pivot 122.

When the latch 118 is disengaged the piston 96 moves under influence of the bias force exerted by the spring 114 to compress the damping liquid 116 and the hydraulic pressure increases. The increase in hydraulic pressure eventually causes the seal 107 to rupture which, in turn, allows damping liquid to flow into the spring chamber 108 and a continued, controlled, movement of the piston 96 to increase the volume of the variable volume fluid receiving space 100 occurs.

In other embodiments the through holes 106a and latch 118 are removed and a rupturable seal 124 (broken line construction in Fig. 7) may be provided to replace, at least in part, the sealed end 112 of the damping chamber 110. On rupture of the seal 124, which in some embodiments may be done manually, damping liquid 116 can leave the damping chamber 110. This causes a reduction in counter-force exerted by the damping liquid 116 and allows the piston 96 to move under the influence of the force exerted by the spring 114.

Other embodiments may include a transport system other than a piston pump system, for example may include a peristaltic pump system, which is fluidly connected to the inlet of a sample test cassette with which it is integrated and which is operable to transport liquid from external of the cassette to elongate lateral flow test strips located in therein.

## Claims

1. A sample test cassette (2; 2a; 2b) comprising an inlet (4; 4a; 4b) for introducing a sample liquid (54) into the sample test cassette (2; 2a; 2b); an elongate channel (8) for receiving an elongate lateral flow test strip (10) and configured with a first end (16) in liquid communication with the inlet (4; 4a; 4b); **characterised in that** the sample test cassette (2; 2a; 2b) further comprises an integral mechanical transport system (12; 92) adapted to generate a flow of sample liquid (54) from outside of the inlet (4; 4a; 4b) and to the first end (16) of the elongate channel (8) via a conduit (14) disposed to also provide liquid communication of the first end (16) with the inlet (4; 4a; 4b) and with the mechanical transport system (12; 92).

2. The sample test cassette (2) as claimed in claim 1 wherein a reservoir (18,20) is provided in liquid communication with the first end (16) of the elongate channel (8) and with the inlet (4) and is adapted to hold sample liquid (54) for contact with a sample receiving portion (204) of an elongate lateral flow test strip (10) received in the elongate channel (8).

3. The sample test cassette as claimed in claim 1 wherein at least a section (32; 36) of a wall (32; 34) of the sample test cassette (2) that overlies at least a portion (8a) of the elongate channel (8) corresponding with an analysis zone (208) of a lateral flow test strip (10) received therein is adapted to allow transmission of optical radiation to and from the portion (8a) of the elongate channel (8).

4. The sample test cassette (2) as claimed in claim 1 wherein the mechanical transport system (12; 92) includes a piston pump assembly having a pump chamber (22; 94) and a piston (24; 96); the piston (24; 96) having a first end (26; 98) slidably engaged with an inner wall (22a; 96a) of the pump chamber (22; 96) to delimit, in cooperation therewith, a variable volume fluid receiving space (28; 100).

5. The sample test cassette (2) as claimed in claim 1 further comprising an elongate lateral flow test strip (10) received in the elongate channel (8).

6. An analyte test system (38) comprising a housing (40); a reading system (48); and a holder (50; 50a; 50b); wherein the holder (50; 50a; 50b) is configured to releasably locate a sample test cassette (2; 2a; 2b) as claimed in any preceding claim in a reading position at which the reading system (48) is aligned in with the elongate channel (8) to permit an interrogation of a test strip (10) located in the elongate channel (8) to test for the presence of an analyte.

7. The analyte test system (38) as claimed in claim 6 wherein the reading system (48) is an optical reading system comprising a complementary light source (48a) and optical detector (48b) arrangement configured to define therebetween an optical path which, when the sample test cassette (2; 2a; 2b) is located in the reading position, intersects the elongate channel (8) to permit an optical interrogation of a test strip (10) located in the elongate channel (8).

8. The analyte test system as claimed in claim 6 wherein the housing (40) comprises a slot (42), for receiving and releasably retaining an own holder (50; 50a; 50b).

9. The analyte test system (38) as claimed in claim 7 wherein one or both of the light source (48a) and the optical detector (48b) is located internal of the holder (50; 50a; 50b).

10. The analyte test system (38) as claimed in claim 6 wherein there is also provided an actuator mechanism (60;80; 114,116 ) adapted to engage with the transport system (12; 92) of a sample test cartridge (2; 2a; 2b) located in the holder (50; 50a; 50b) and to actuate the transport system (12; 92) to generate the flow of sample liquid (54).

11. The analyte test system (38) as claimed in claim 10 wherein the holder (50) holds internally an own actuator mechanism (60; 80).

12. The analyte test system (38) as claimed claim 10 or claim 11 wherein the actuator mechanism (60; 80) comprises a drive (66; 88) engagable with the transport system (12) and rotatable to actuate the transport system (12) to generate the flow of sample liquid (54).

13. The analyte test system (38) as claimed in claim 12 wherein the drive comprises a rotatable disc (66) and an arm (62) having a first end (64) fixed to the rotatable disc (66) and a second end (70) configured with a detent (68) adapted to releasably mechanically engage with the transport system (12).

14. The analyte test system (38) as claimed in claim 13 wherein a protrusion (78) is formed on the rotatable disc (66) circumferentially displaced on the rotatable disc (66) from the first end (64) of the arm (62) to contact the arm (62) when the rotatable disc (66) is rotated by a predetermined amount.

15. The analyte test system (38) as claimed in any of the claims 13 or 14 wherein the transport system (12) comprises a pump chamber (22) arranged in liquid communication with an end of the conduit (14); and a piston (24) having a first end (26) slidably engaged with an inner wall (22a) of the pump chamber (22) to delimit therewith a variable volume fluid receiving space (28) and having a second end (30) provided with a surface (72) for releasably mechanically engaging with the detent (68).

16. The analyte test system (38) as claimed in claim 12 wherein the drive comprises a sprocket (88) engagable with a toothed portion (84) of a piston (86) of the transport system (12) and being rotatable to impart a linear motion to the piston (86) when engaged with the toothed portion (84).

17. The analyte test system (38) as claimed in claim 10 wherein the sample cassette (2) holds the actuator mechanism (114,116) housed in a compartment (104) divided internally by a second end (106) of a piston (96) into a spring chamber, housing a spring (114) provided in engagement with the second end (106) and a damping chamber (110) housing a damping fluid (116), the second end (106) being distal a first end (98) of the piston (96) which is located in slidable engagement with an inner wall 94a of a pump chamber (94).

## Patentansprüche

1. Probentestkassette (2; 2a; 2b), umfassend einen Einlass (4; 4a; 4b) zum Einbringen einer Probenflüssigkeit (54) in die Probentestkassette (2; 2a; 2b); einen länglichen Kanal (8) zum Aufnehmen eines länglichen Lateral-Flow-Test-Streifens (10), und der mit einem ersten Ende (16) in flüssiger Kommunikation mit dem Einlass (4; 4a; 4b) konfiguriert ist; **dadurch gekennzeichnet, dass** die Probentestkassette (2; 2a; 2b) ferner ein integriertes mechanisches Transportsystem (12; 92) umfasst, das geeignet ist, um einen Fluss von Probenflüssigkeit (54) von außerhalb des Einlasses (4; 4a; 4b) und zu dem ersten Ende (16) des länglichen Kanals (8) über eine Leitung (14) zu erzeugen, die eingerichtet ist, um ebenso eine flüssige Kommunikation des ersten Endes (16) mit dem Einlass (4; 4a; 4b) und mit dem mechanischen Transportsystem (12; 92) bereitzustellen.

2. Probentestkassette (2) nach Anspruch 1, wobei ein Behälter (18,20) in flüssiger Kommunikation mit dem ersten Ende (16) des länglichen Kanals (8) und mit dem Einlass (4) bereitgestellt ist, und geeignet ist, um Probenflüssigkeit (54) für eine Berührung mit einem Probenaufnahmeabschnitt (204) eines länglichen Lateral-Flow-Test-Streifens (10), der in dem länglichen Kanal (8) aufgenommen ist, zu halten.

3. Probentestkassette nach Anspruch 1, wobei mindestens ein Bereich (32; 36) einer Wand (32; 34) der Probentestkassette (2), der über mindestens einem Abschnitt (8a) des länglichen Kanals (8) liegt, der einer Analysezone (208) des Lateral-Flow-Test-Streifens (10) entspricht, der darin aufgenommen ist, geeignet ist, um eine Übertragung von optischer Strahlung zu und von dem Abschnitt (8a) des länglichen Kanals (8) zu ermöglichen.

4. Probentestkassette (2) nach Anspruch 1, wobei das mechanische Transportsystem (12; 92) eine Kolbenpumpenbaugruppe einschließt, die eine Pumpenkammer (22; 94) und einen Kolben (24; 96) aufweist; wobei der Kolben (24; 96) ein erstes Ende (26; 98) aufweist, das mit einer Innenwand (22a; 96a) der Pumpenkammer (22; 96) verschiebbar in Eingriff steht, um in Zusammenwirkung damit einen Fluidaufnahmeraum (28; 100) mit variablem Volumen zu begrenzen.

5. Probentestkassette (2) nach Anspruch 1, ferner umfassend einen länglichen Lateral-Flow-Test-Streifen (10), der in dem länglichen Kanal (8) aufgenommen ist.

6. Analyt-Testsystem (38), umfassend ein Gehäuse (40); ein Lesesystem (48); und einen Halter (50; 50a; 50b); wobei der Halter (50; 50a; 50b) konfiguriert ist, um eine Probentestkassette (2; 2a; 2b) nach einem der vorstehenden Ansprüche in einer Leseposition lösbar zu fixieren, an der das Lesesystem (48) in dem länglichen Kanal (8) ausgerichtet ist, um eine Abfrage eines Teststreifens (10) zu erlauben, der sich in dem länglichen Kanal (8) befindet, um auf das Vorhandensein eines Analyten zu testen.

7. Analyt-Testsystem (38) nach Anspruch 6, wobei das Lesesystem (48) ein optisches Lesesystem ist, umfassend eine komplementäre Lichtquelle (48a) und eine optische Detektor(48b)-Anordnung, die konfiguriert ist, um dazwischen einen Strahlengang zu definieren, der, wenn sich die Probentestkassette (2; 2a; 2b) in der Leseposition befindet, den länglichen Kanal (8) schneidet, um eine optische Abfrage eines Teststreifens (10) zu erlauben, der sich in dem länglichen Kanal (8) befindet.

8. Analyt-Testsystem nach Anspruch 6, wobei das Gehäuse (40) einen Schlitz (42) zum Aufnehmen und lösbaren Halten eines eigenen Halters (50; 50a; 50b) umfasst.

9. Analyt-Testsystem (38) nach Anspruch 7, wobei eine oder beide der Lichtquelle (48a) und der optische Detektor (48b) sich im Inneren des Halters (50; 50a; 50b) befinden.

10. Analyt-Testsystem (38) nach Anspruch 6, wobei ebenso ein Aktuatormechanismus (60;80; 114,116) bereitgestellt ist, der geeignet ist, um mit dem Transportsystem (12; 92) einer Probentestpatrone (2; 2a; 2b) in Eingriff zu stehen, die sich in dem Halter (50; 50a; 50b) befindet, und um das Transportsystem (12; 92) zu betätigen, um den Fluss der Probenflüssigkeit (54) zu erzeugen.

11. Analyt-Testsystem (38) nach Anspruch 10, wobei der Halter (50) einen eigenen Aktuatormechanismus (60; 80) im Inneren hält.

12. Analyt-Testsystem (38) nach Anspruch 10 oder 11, wobei der Aktuatormechanismus (60; 80) einen Antrieb (66; 88) umfasst, der mit dem Transportsystem (12) in Eingriff gebracht werden kann und drehbar ist, um das Transportsystem (12) zu betätigen, um den Fluss der Probenflüssigkeit (54) zu erzeugen.

13. Analyt-Testsystem (38) nach Anspruch 12, wobei der Antrieb eine drehbare Scheibe (66) und einen Arm (62) umfasst, der ein erstes Ende (64), das an der drehbaren Scheibe (66) befestigt ist, und ein zweites Ende (70), das mit einer Verrastung (68) konfiguriert ist, die geeignet ist, um mit dem Transportsystem (12) lösbar mechanisch in Eingriff zu kommen, aufweist.

14. Analyt-Testsystem (38) nach Anspruch 13, wobei ein Vorsprung (78) auf der drehbaren Scheibe (66) ausgebildet ist, der auf der drehbaren Scheibe (66) von dem ersten Ende (64) des Arms (62) in Umfangsrichtung verschoben ist, um den Arm (62) zu berühren, wenn die drehbare Scheibe (66) um einen vorbestimmten Betrag gedreht wird.

15. Analyt-Testsystem (38) nach einem der Ansprüche 13 oder 14, wobei das Transportsystem (12) eine Pumpenkammer (22), die in flüssiger Kommunikation mit einem Ende der Leitung (14) angeordnet ist; und einen Kolben (24), der ein erstes Ende (26) aufweist, das mit einer Innenwand (22a) der Pumpenkammer (22) verschiebbar in Eingriff steht, um damit einen Fluidaufnahmeraum (28) mit variablem Volumen zu begrenzen, und ein zweites Ende (30) aufweist, das mit einer Oberfläche (72) zum lösbar mechanischen Eingreifen mit der Klinke (68) versehen ist, umfasst.

16. Analyt-Testsystem (38) nach Anspruch 12, wobei der Antrieb ein Zahnrad (88) umfasst, das mit einem gezahnten Abschnitt (84) eines Kolbens (86) des Transportsystems (12) in Eingriff gebracht werden kann und drehbar ist, um dem Kolben (86) eine lineare Bewegung zu verleihen, wenn es mit dem gezahnten Abschnitt (84) in Eingriff steht.

17. Analyt-Testsystem (38) nach Anspruch 10, wobei die Probenkassette (2) den Aktuatormechanismus (114,116) hält, der in einem Fach (104) aufgenommen ist, das intern durch ein zweites Ende (106) eines Kolbens (96) in eine Federkammer unterteilt ist, die eine Feder (114) aufnimmt, die in Eingriff mit dem zweiten Ende (106) bereitgestellt ist, und eine Dämpfungskammer (110), die ein Dämpfungsfluid (116) aufnimmt, wobei das zweite Ende (106) distal zu einem ersten Ende (98) des Kolbens (96) ist, der sich in verschiebbarem Eingriff mit einer Innenwand 94a einer Pumpenkammer (94) befindet.

## Revendications

1. Cassette de test d'échantillon (2 ; 2a ; 2b) comprenant une entrée (4 ; 4a ; 4b) permettant d'introduire un liquide échantillon (54) dans la cassette de test d'échantillon (2 ; 2a ; 2b) ; un canal allongé (8) permettant de recevoir une bande de test à flux latéral allongée (10) et conçue avec une première extrémité (16) en communication liquide avec l'entrée (4 ; 4a ; 4b) ; **caractérisée en ce que** la cassette de test d'échantillon (2 ; 2a ; 2b) comprend en outre un système de transport mécanique intégral (12 ; 92) conçu pour générer un flux de liquide échantillon (54) depuis l'extérieur de l'entrée (4 ; 4a ; 4b) et vers la première extrémité (16) du canal allongé (8) par l'intermédiaire d'un conduit (14) disposé pour fournir également une communication liquide de la première extrémité (16) avec l'entrée (4 ; 4a ; 4b) et avec le système de transport mécanique (12 ; 92).

2. Cassette de test d'échantillon (2) selon la revendication 1, dans laquelle un réservoir (18, 20) est prévu en communication liquide avec la première extrémité (16) du canal allongé (8) et avec l'entrée (4) et est adapté pour maintenir un liquide d'échantillon (54) pour entrer en contact avec une partie de réception d'échantillon (204) d'une bande de test à flux latéral allongée (10) reçue dans le canal allongé (8).

3. Cassette de test d'échantillon selon la revendication 1, dans laquelle au moins une section (32 ; 36) d'une paroi (32 ; 34) de la cassette de test d'échantillon (2) qui recouvre au moins une partie (8a) du canal allongé (8) correspondant à une zone d'analyse (208) d'une bande de test à flux latéral (10) reçue à l'intérieur de celle-ci est adaptée pour permettre une transmission de rayonnement optique vers et depuis la partie (8a) du canal allongé (8).

4. Cassette de test d'échantillon (2) selon la revendication 1, dans laquelle le système de transport mécanique (12 ; 92) comporte un ensemble pompe à piston ayant une chambre de pompe (22 ; 94) et un piston (24 ; 96) ; le piston (24 ; 96) ayant une première extrémité (26 ; 98) mis en prise de manière coulissante avec une paroi interne (22a ; 96a) de la chambre de pompe (22 ; 96) pour délimiter, en coopération avec celle-ci, un espace de réception de fluide à volume variable (28 ; 100).

5. Cassette de test d'échantillon (2) selon la revendication 1, comprenant en outre une bande de test à flux latéral allongée (10) reçue dans le canal allongé (8).

6. Système de test d'analyte (38) comprenant un logement (40) ; un système de lecture (48) ; et un dispositif de maintien (50 ; 50a ; 50b) ; dans lequel le dispositif de maintien (50 ; 50a ; 50b) est conçu pour situer de manière libérable une cassette de test d'échantillon (2 ; 2a ; 2b) selon l'une quelconque revendication précédente dans une position de lecture au niveau de laquelle le système de lecture (48) est aligné sur le canal allongé (8) pour permettre une interrogation d'une bande de test (10) située dans le canal allongé (8) pour tester la présence d'un analyte.

7. Système de test d'analyte (38) selon la revendication 6 dans lequel le système de lecture (48) est un système de lecture optique comprenant un agencement de source de lumière complémentaire (48a) et de détecteur optique (48b) configurés pour définir entre eux un trajet optique qui, lorsque la cassette de test d'échantillon (2 ; 2a ; 2b) est située dans la position de lecture, coupe le canal allongé (8) pour permettre une interrogation optique d'une bande de test (10) située dans le canal allongé (8).

8. Système de test d'analyte selon la revendication 6, dans lequel le logement (40) comprend une fente (42), pour recevoir et retenir de manière libérable un dispositif de maintien propre (50 ; 50a ; 50b).

9. Système de test d'analyte (38) selon la revendication 7, dans lequel l'un et/ou l'autre parmi la source de lumière (48a) et le détecteur optique (48b) est situé interne au dispositif de maintien (50 ; 50a ; 50b).

10. Système de test d'analyte (38) selon la revendication 6, dans lequel il est également prévu un mécanisme actionneur (60 ; 80 ; 114, 116) adapté pour venir en prise avec le système de transport (12 ; 92) d'une cartouche de test d'échantillon (2 ; 2a ; 2b) située dans le dispositif de maintien (50 ; 50a ; 50b) et pour actionner le système de transport (12 ; 92) pour générer le flux de liquide échantillon (54).

11. Système de test d'analyte (38) selon la revendication 10, dans lequel le dispositif de maintien (50) maintient à l'intérieur un mécanisme actionneur propre (60 ; 80).

12. Système de test d'analyte (38) selon la revendication 10 ou la revendication 11, dans lequel le mécanisme actionneur (60 ; 80) comprend un entraînement (66 ; 88) pouvant venir en prise avec le système de transport (12) et pouvant tourner pour actionner le système de transport (12) pour générer le flux de liquide échantillon (54).

13. Système de test d'analyte (38) selon la revendication 12, dans lequel l'entraînement comprend un disque rotatif (66) et un bras (62) ayant une première extrémité (64) fixée au disque rotatif (66) et une seconde extrémité (70) conçue avec un cran (68) adapté pour venir en prise mécaniquement de manière libérable avec le système de transport (12).

14. Système de test d'analyte (38) selon la revendication 13, dans lequel une saillie (78) est formée sur le disque rotatif (66) circonférentiellement déplacé sur le disque rotatif (66) depuis la première extrémité (64) du bras (62) pour entrer en contact avec le bras (62) lorsque le disque rotatif (66) est tourné d'une quantité prédéterminée.

15. Système de test d'analyte (38) selon l'une quelconque des revendications 13 ou 14, dans lequel le système de transport (12) comprend une chambre de pompe (22) agencée en communication liquide avec une extrémité du conduit (14); et un piston (24) ayant une première extrémité (26) mise en prise de manière coulissante avec une paroi interne (22a) de la chambre de pompe (22) pour délimiter avec celle-ci un espace de réception de fluide à volume variable (28) et ayant une seconde extrémité (30) pourvue d'une surface (72) pour venir en prise mécaniquement de manière libérable avec le cran (68).

16. Système de test d'analyte (38) selon la revendication 12, dans lequel l'entraînement comprend une roue dentée (88) pouvant être mis en prise avec une partie dentée (84) d'un piston (86) du système de transport (12) et pouvant tourner pour communiquer un mouvement linéaire au piston (86) lorsqu'il est mis en prise avec la partie dentée (84).

17. Système de test d'analyte (38) selon la revendication 10, dans lequel la cassette d'échantillon (2) maintient le mécanisme actionneur (114, 116) logé dans un compartiment (104) divisé intérieurement par une seconde extrémité (106) d'un piston (96) dans une chambre à ressort, logeant un ressort (114) prévu en prise avec la seconde extrémité (106) et une chambre d'amortissement (110) logeant un fluide d'amortissement (116), la seconde extrémité (106) étant distale d'une première extrémité (98) du piston (96) qui est située en prise coulissante avec une paroi interne 94a d'une chambre de pompe (94).
